# EUROPEAN PATENT SPECIFICATION

(11) **EP 4 457 775 B1**
(45) Date of publication and mention of the grant of the patent: **12.11.2025**
(21) Application number: 22840787.0
(22) Date of filing: 30.12.2022
(51) Int. Cl.: G06V 10/77

(54) **METHOD AND DEVICE FOR NON-INVASIVE TOMOGRAPHIC CHARACTERISATION OF A SAMPLE COMPRISING A PLURALITY OF DIFFERENTIATED TISSUES**
VERFAHREN UND VORRICHTUNG ZUR NICHTINVASIVEN TOMOGRAPHISCHEN CHARAKTERISIERUNG EINER PROBE MIT EINER VIELZAHL VON DIFFERENZIERTEN GEWEBEN
PROCÉDÉ ET DISPOSITIF DE CARACTÉRISATION TOMOGRAPHIQUE NON INVASIVE D'UN ÉCHANTILLON COMPRENANT UNE PLURALITÉ DE TISSUS DIFFÉRENCIÉS

(30) Priority: 31.12.2021 PT 2021117727; 05.01.2022 PT 2022117732
(43) Date of publication of application: 06.11.2024
(73) Proprietor: INESC TEC - Instituto de Engenharia de Sistemas e Computadores, Tecnologia e Ciência, 4200-465 Porto (PT); Universidade Do Porto, 4099-002 Porto (PT)
(72) Inventor: DA COSTA MARTINS, Rui Miguel, 4200-465 PORTO (PT); BAPTISTA NEVES DOS SANTOS, Filipe, 4200-465 PORTO (PT); DE MIRANDA FURTADO CAMPOS CUNHA, Mário Manuel, 4200-465 PORTO (PT); MARQUES MONTEIRO DA SILVA, Aníbal Filipe, 4200-465 PORTO (PT); TOSIN, Renan, 4200-465 PORTO (PT); COSTA MAGALHÃES, Sandro Augusto, 4200-465 PORTO (PT); REIS PEREIRA, Mafalda, 4200-465 Porto (PT)
(74) Representative: Patentree
(86) International application number: PCT/EP2022/088102
(87) International publication number: WO 2023/126532

(56) References cited:
- CN-A- 101 196 491
- US-A1- 2019 004 035
- BERZIN I ET AL: "A NON-DESTRUCTIVE METHOD FOR SECONDARY METABOLITE DETERMINATION IN HAIRY ROOT CULTURES", JOURNAL OF CHEMICAL ENGINEERING OF JAPAN, SOCIETY OF CHEMICAL ENGINEERS, JP, vol. 32, no. 2, 1 April 1999 (1999-04-01), pages 229 - 234, XP000824389, ISSN: 0021-9592, DOI: 10.1252/JCEJ.32.229
- NICOLAI ET AL: "Nondestructive measurement of fruit and vegetable quality by means of NIR spectroscopy: A review", POSTHARVEST BIOLOGY AND TECHNOLOGY, ELSEVIER, AMSTERDAM, NL, vol. 46, no. 2, 28 September 2007 (2007-09-28), pages 99 - 118, XP022277290, ISSN: 0925-5214, DOI: 10.1016/J.POSTHARVBIO.2007.06.024
- QIN J ET AL: "Measurement of the optical properties of fruits and vegetables using spatially resolved hyperspectral diffuse reflectance imaging technique", POSTHARVEST BIOLOGY AND TECHNOLOGY, ELSEVIER, AMSTERDAM, NL, vol. 49, no. 3, 1 September 2008 (2008-09-01), pages 355 - 365, XP022733506, ISSN: 0925-5214, [retrieved on 20080513], DOI: 10.1016/J.POSTHARVBIO.2008.03.010

## Description

### TECHNICAL FIELD

The present disclosure relates to a method and device for non-invasive tomographic characterisation of a sample comprising a plurality of differentiated tissues.

### BACKGROUND

Mainstream analytical chemistry and biosensor approaches to spectroscopy information complexity are to use physical separation unit operations [1,2], reaction-specific labelling [3,4], or more recently label-free reaction specificity [5]. State-of-the-art chemometrics makes use of macroscopic hyperspectral or multispectral imaging to relate surface characteristics of plant tissues (e.g., fruits or leaves) to pigmentation and disease symptoms [6]. The previous art methods are cumbersome to be used *in vivo,* particularly in an open field context:
i. Biosensors need destructive sampling, consumables, provide limited sampling or even need sample preparation, all of which make them impractical to be implemented on robotic platforms for high-throughput monitoring of vast agricultural areas;
ii. Hyperspectral cameras capture a picture in a wavelength interval of light reflected from the plant surface. The reflected light is non-absorbed light; therefore, it carries little information about the sample composition, with insufficient information relating to omics quantitative information.

Document US2019004035A1 discloses a method for determining a metabolic state of a plant or part thereof comprising a) rapid evaporating a multitude of metabolites of said plant or part thereof; b) determining the amount of at least one metabolite characteristic of said metabolic state; and c) thereby, determining a metabolic state of a plant thereof. Further provided is a method for in vivo determining a metabolite distribution in a plant or part thereof comprising a) in vivo rapid evaporating at least one metabolite of interest in at least a first and a second location of said plant or part thereof; b) determining the amounts of at least one metabolite at said first and a second location; and, c) thereby, in vivo determining metabolite distribution in a plant or part thereof. Moreover, provided are devices, data collections, and uses relating to the aforesaid methods.

These facts are disclosed in order to illustrate the technical problem addressed by the present disclosure.

### GENERAL DESCRIPTION

The invention is defined by a computer-implemented method for non-invasive tomographic metabolite characterisation of a vegetable or animal sample to be characterised, as defined in claim 1.

The invention is also defined by a device for non-invasive tomographic metabolite characterisation of a vegetable or animal sample to be characterised, as stated in claim 11.

The present document discloses a computer-implemented method for non-invasive tomographic metabolite characterisation of a vegetable or animal sample to be characterised, the sample comprising a plurality of differentiated tissues from a set of previously-characterised differentiated tissues, by using a multidimensional latent structure model of differentiated tissues, which was previously obtained by: scanning tissue samples belonging to each differentiated tissue of the set with a hyperspectral microscope and spectrometer; acquiring hyperspectral spectrum or spectra for each differentiated tissue sample from the hyperspectral microscope and spectrometer; analysing the same tissue samples for the metabolites to be characterised; calculating the latent structure model from the acquired spectra and analysed metabolites; the method comprising the steps of: spatially scanning the sample to be characterised with a hyperspectral point-of-measurement, POM, optical device and a spectrometer; acquiring hyperspectral spectrum or spectra from each of a plurality of spatial positions and/or orientations of the POM optical device and spectrometer in respect of the sample to be characterised; using the previously obtained model to deconvolute the metabolites to be characterised for each of the differentiated tissues from the acquired spectra.

In an embodiment, the scanning of tissue samples belonging to each tissue of the set with a hyperspectral microscope and spectrometer is microscopic, and the spatially scanning the sample to be characterised with a hyperspectral POM optical device and spectrometer is macroscopic.

In an embodiment, the spatially scanning comprises varying one or more of the following to obtain a spatial diversity of the POM spatial scanning: displacement of the POM optical device; point-of-view angle of the POM optical device; different scales of magnification of the POM optical device; moving the POM optical device lines of sight to cause a parallax effect.

According to the invention, the multidimensional latent structure model is a hierarchical latent structure model.

According to the invention, the hierarchical latent structure model comprises a latent variable model comprising a latent variable super-space and corresponding latent variable sub-spaces.

According to the invention, the hierarchical relationship of the hierarchical latent structure model comprises super-space and corresponding sub-spaces: a latent sub-space for spectral data of each of the differentiated tissues; a super-space for each of the metabolites to be characterised, translating the combination of hyperspectral data to the metabolites to be characterised.

In an embodiment, the sample to be characterised is a three-dimensional tissue structure or organ.

In an embodiment, the metabolites to be characterised comprise pigments, tannins, photosynthesis, energy storage, nutrient uptake, necrosis, infection probability, or combinations thereof.

In an embodiment, the sample to be characterised is a vegetable sample, in particular the sample to be characterised is a tomato or grape tissue sample.

In an embodiment, the sample to be characterised is vegetable sample and the differentiated tissues comprise skin, pulp, and seed tissue.

In an embodiment, the scanning of tissue samples belonging to each differentiated tissue is carried out along a sampling grid.

In an embodiment, the scanning of tissue samples belonging to each differentiated tissue is further carried out along discrete sampling depths.

In an embodiment, the spectra are UV-VIS-NIR.

It is further disclosed a device for non-invasive tomographic metabolite characterisation of a vegetable or animal sample to be characterised, the sample comprising a plurality of differentiated tissues from a set of previously-characterised differentiated tissues, including a computer processor and a non-transitory computer-readable medium comprising a multidimensional latent structure model of differentiated tissues, which was previously obtained by: scanning tissue samples belonging to each differentiated tissue of the set with a hyperspectral microscope and spectrometer; acquiring hyperspectral spectrum or spectra for differentiated each tissue sample from the microscope and spectrometer; analysing the same tissue samples for the metabolites to be characterised; calculating the latent structure model from the acquired spectra and analysed metabolites; wherein the computer processor is configured for: spatially scanning the sample to be characterised with a hyperspectral point-of-measurement, POM, optical device and spectrometer; acquiring hyperspectral spectra from a plurality of spatial positions and/or orientations of the POM optical device and spectrometer in respect of the sample to be characterised; using the previously obtained model to deconvolute the metabolites to be characterised for each of the differentiated tissues from the acquired spectra.

It is also presented a non-transitory computer-readable medium comprising computer program instructions for implementing a device for non-invasive tomographic metabolite characterisation of a vegetable or animal sample to be characterised, which when executed by a processor, cause the processor to carry out the disclosed method.

One of the present disclosure aims is to develop a non-invasive spectroscopy tomography system for compositional imaging of plant and animal tissues. This technology is demonstrated in application to precision agriculture and plant physiology. The present disclosure is based on image reconstruction using latent hierarchical information fusion methods to unscramble the observed spectroscopy signal into its different components of the observed tridimensional structures, particularly the different plant tissues being recorded, such as in grapes the skin, pulp, and seeds. Hierarchical relations exploit the parallax effect, which changes the spectral fingerprint depending upon the point-of-view angle of the spectroscopy probe. Reconstruction is performed by the hierarchical latent relationships between spectral patterns of the observed tissues. This principle is used to develop:
i. macroscopic tomography - for measuring macroscopic properties of plant internal tissues with case studies of grape and tomato fruits (e.g., skin, pulp, and seeds);
ii. microscopic tomography - for measuring microscopic spectral patterns that can provide a diagnosis at very early stages, e.g., of tomato leaf bacterial infection by *Pseudomonas syringae* and *Xanthomonas euvesicatoria*; and
iii. Multi-scaled tomography results - exploiting the hierarchical parallax relationship between latent spaces of different scales of magnification to provide microscopic reconstructions based on macroscopic measurements.

Results show the feasibility of relationship reconstruction from latent structures, as well as, providing relationship between macroscopic and microscopic spectral data.

Spectral data carries both physical and chemical information. The observed spectrum of plant tissue is the superposition of all structures in the field of view, scattering, matrix effects, and multi-scaled interference. Being able to use these complex interactions is paramount to unscrambling the information present in each recorded spectrum. The complexity of spectral data has been the foremost hurdle to the existence of *in vivo,* reagent-less, and non-invasive chemical or metabolic diagnosis.

The macroscopic spectra are recorded and related to the hyperspectral image by their corresponding latent space.

The capacity of internal structures reconstruction, compositional, and disease diagnosis at very early stages centres molecular biology and plant physiology as the main driver of information in precision agriculture and plant biotechnology. The present disclosure bridges the information between *in vitro* laboratory and *in vivo* field studies by allowing high-throughput and multi-scaled monitoring of plants in their ecosystem without disrupting the interaction of plants with soil, climate, and ecosystem. Allowing information transfer between the field and laboratory tests is a critical enabling transformation in precision agriculture that allows evaluating to be much faster, thus promoting scientific advances in both phytopharmaceuticals and agricultural practices for early-stage and low-impact practices.

One of the main objectives of the disclosure is to provide a macroscopic, microscopic, and multi-scaled spectral reconstruction, compositional, and infection diagnosis through spectroscopy POM devices and advanced signal/information processing, demonstrating the feasibility of tomographic reconstruction by hierarchical latent structures methods.

### BRIEF DESCRIPTION OF THE DRAWINGS

The following figures provide preferred embodiments for illustrating the disclosure. They should not be seen as limiting the scope of the invention.

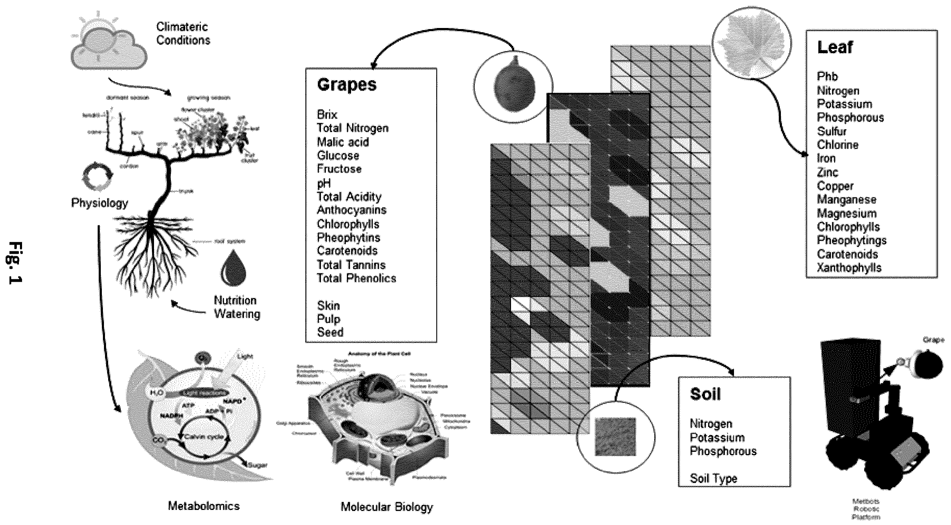

**Figure 1****:** Schematic representation of an embodiment of a device, robotic platform and metabolic quantification according to the disclosure.

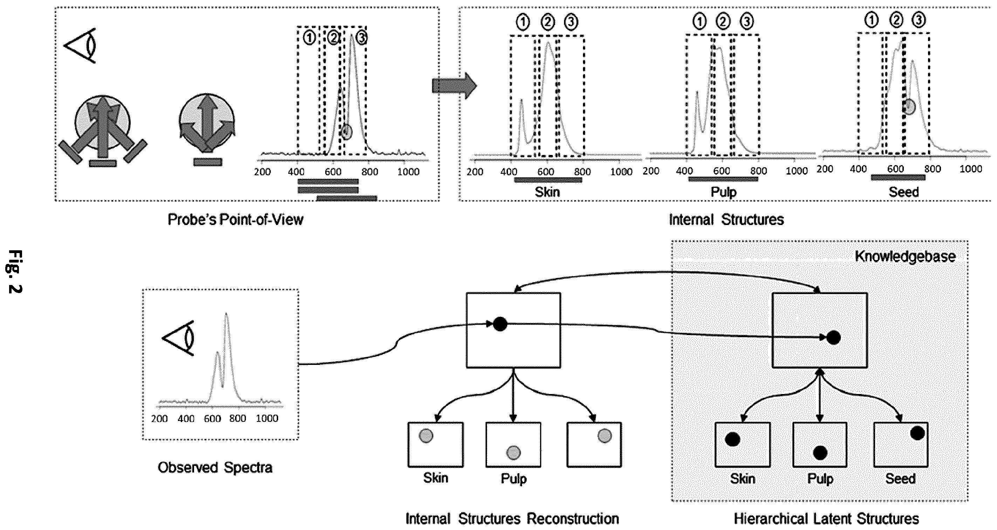

**Figure 2****:** Schematic representation of an embodiment of 3D internal spectroscopy reconstruction method using hierarchical latent spaces.

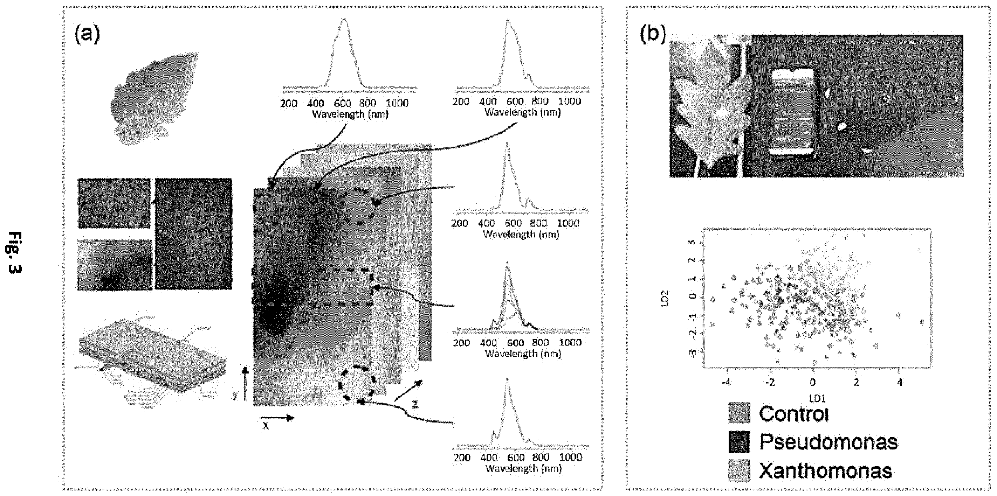

**Figure 3****:** Schematic representation of an embodiment of hyperspectral microscopy tomography and relationship to macroscopic measurements: (a) hyperspectral image data of infected leaf along different depths (z) where the extension of the infected lesions is explicit in spectral patterns; and (b) macroscopic measurements with device and corresponding classification of infection using linear discriminant analysis (LDA) to spectral data.

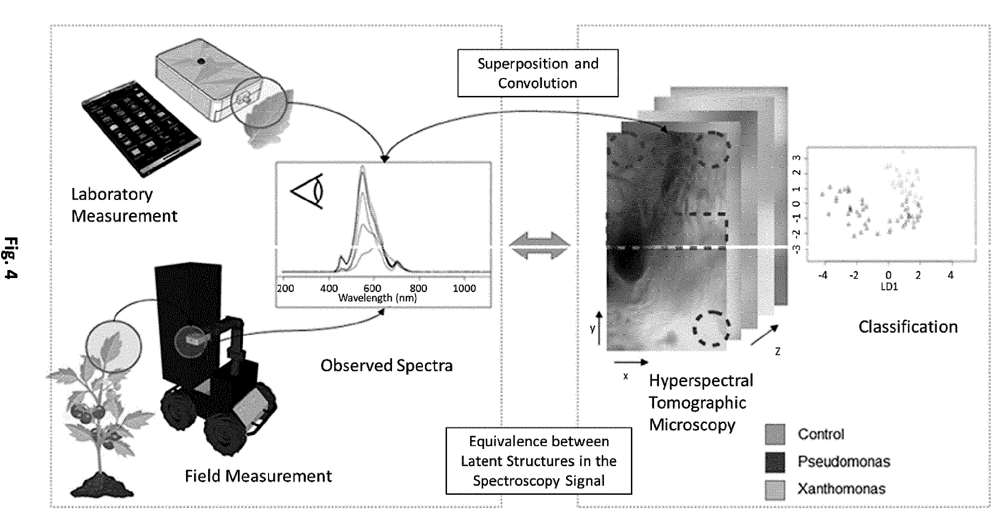

**Figure 4****:** Schematic representation of an embodiment of multi-scaled hyperspectral tomography reconstruction of latent structures equivalence of information as the macroscopic information is the superposition and convolution of the microscopic information, resulting to less abrupt changes in the macroscopic spectra that once related to the micro-scale information open the possibility of non-invasive diagnosis by simple optical adaptations to existing hardware.

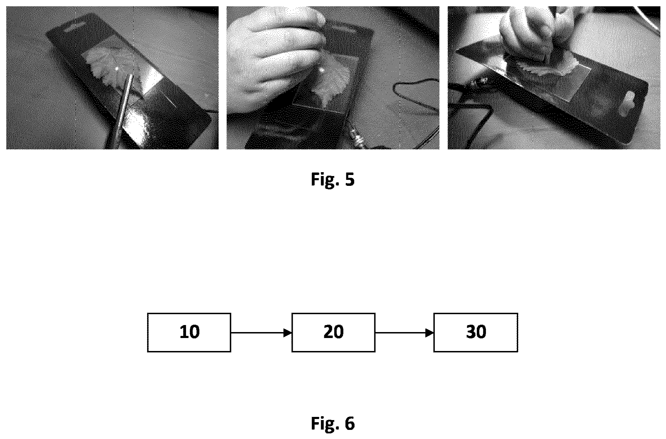

**Figure 5****:** Schematic representation of an embodiment of macroscopical setup consisting of a manual probe to acquire data on select matrix spots.
**Figure 6****:** Schematic representation of an embodiment of hyperspectral microscope setup composed by a Zeiss Axiovert.A1 **10,** connected to an Ocean Insight HR4000 spectrometer **30** via an UV-Vis-NIR 200 µm (I.D.) optical fiber **20,** fine-tuned for accurate, simultaneous spectral acquisition of a sampled micrograph.

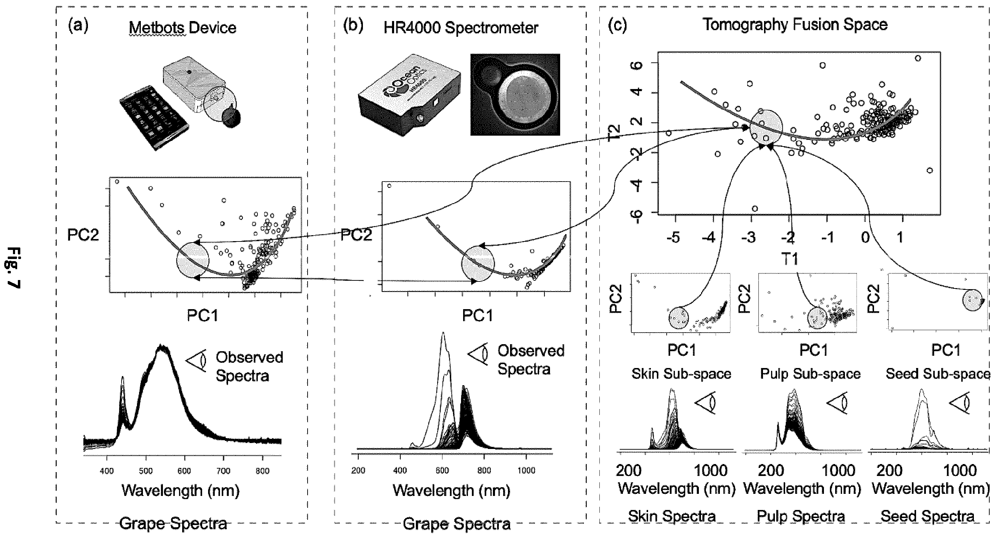

**Figure 7****:** Schematic representation of an embodiment of macroscopic tomography in grapes: measurements made with a robotic device and HR4000 spectrometer to the hole grape; correspondence between spectral variance space of the robotic device and HR4000 spectrometer and its relationship to the tomography fusion space, obtained by using the information from skin, pulp and seed variance sub-spaces.

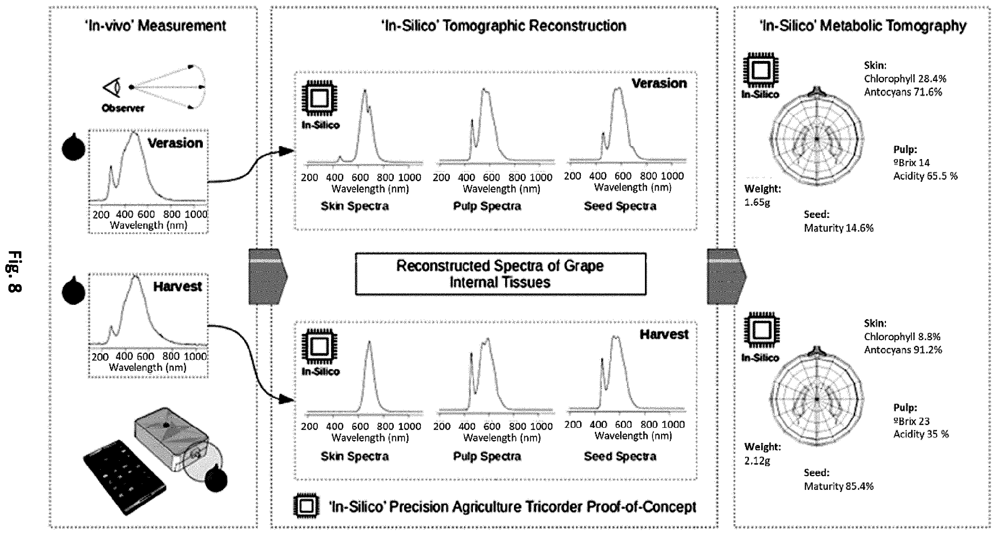

**Figure 8****:** Schematic representation of an embodiment of macroscopic tomography reconstruction: 'in- vivo' measurement of two different grapes at veraison and near harvest time, corresponding 'in- silico' reconstruction of the skin, pulp and seed tissues spectra and estimated relevant properties of each tissue.

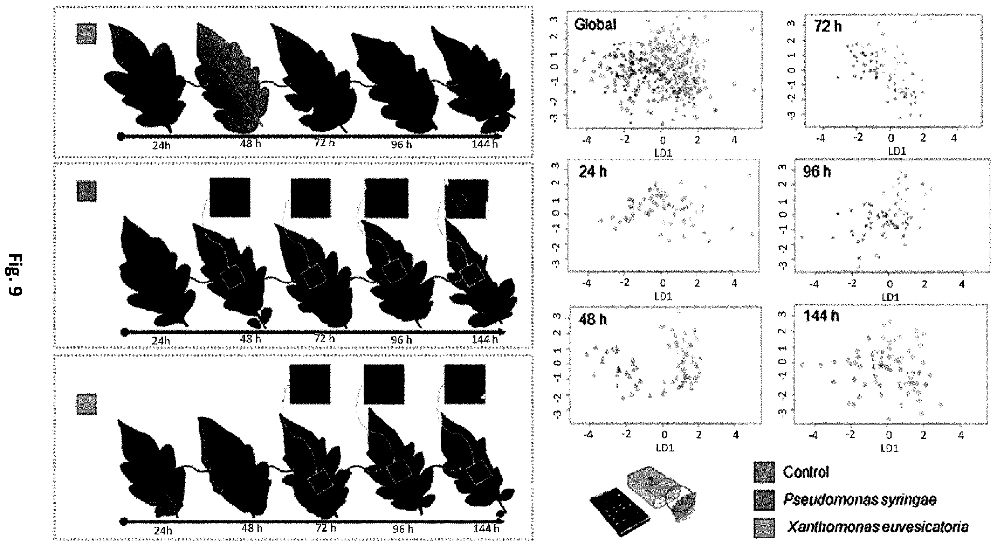

**Figure 9****:** Schematic representation of an embodiment of macroscopic results: visual inspection to control and inoculated leaves with *Pseudomonas syringae* and *Xanthomonas euvesicatoria* infection progression (left), and corresponding spectral classification using linear discriminant analysis from 24 h to 144 h after inoculation (right).

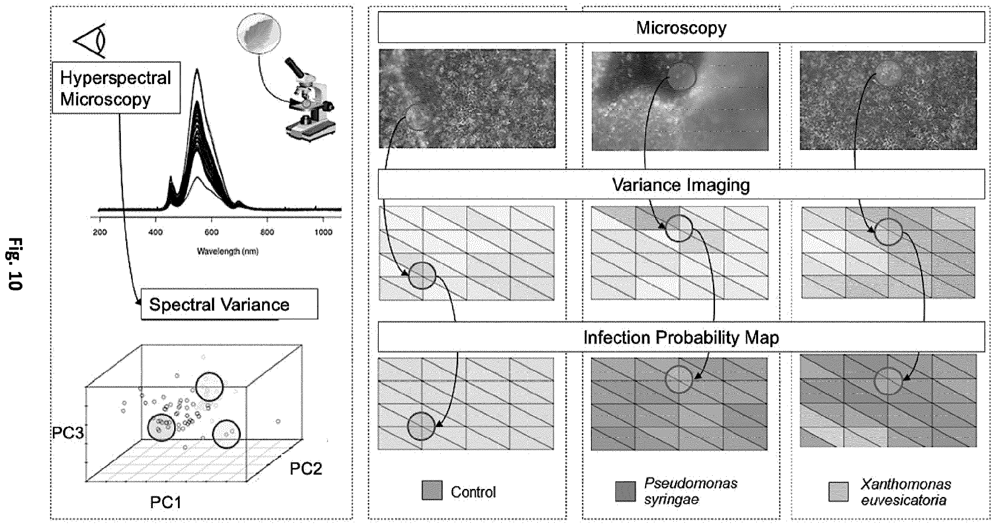

**Figure 10****:** Schematic representation of an embodiment of microscopic results: observed spectra at 144 h at the hyperspectral microscope (200×), principal component analysis of spectral signal and infection classification, microscope images and corresponding hyperspectral analysis by variance imaging and the corresponding probability of infection by partial least squares regression discriminant analysis.

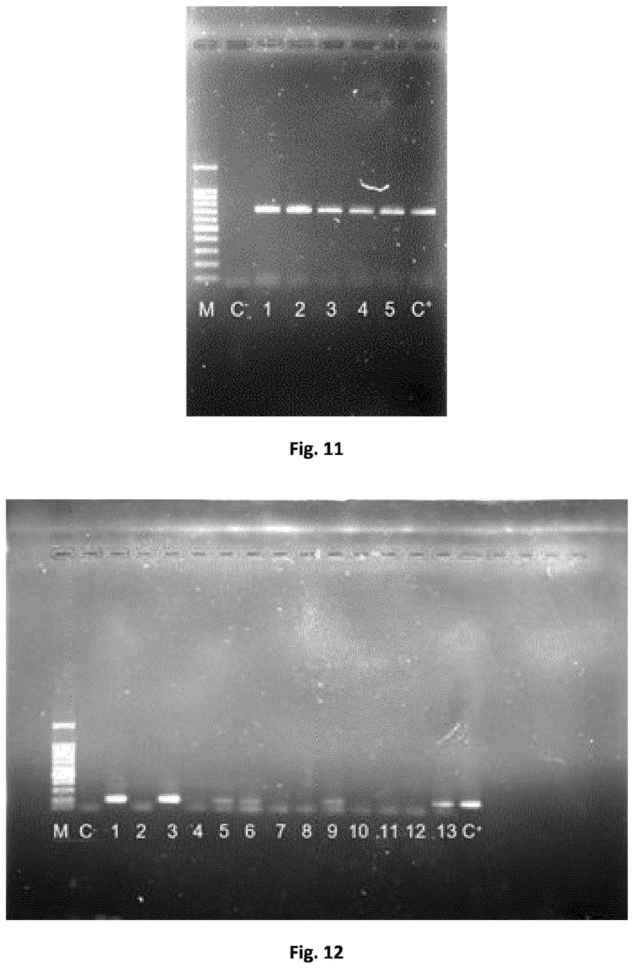

**Figure 11****:** Schematic representation of an embodiment of colony PCR analysis of infected tomato leaves with DNA markers XV14 (713 bp) for detection of *Xanthomonas euvesicatoria* LMG 905. C- - Colony PCR using distilled water as template. 1, 2, 3 - DNA samples from different bacterial colonies were obtained through pathogen isolation assay performed 24 h after infection. 4, 5 - DNA samples from different bacterial colonies obtained through pathogen isolation assay performed 48 h after infection. C+ - *Xanthomonas euvesicatoria* DNA stored at -80 ºC belonging to the laboratory bacterial collection.
**Figure 12****:** Schematic representation of an embodiment of colony PCR analysis of infected tomato leaves with DNA markers PST2 (200 bp) for detection of *Pseudomonas syringae* pv. tomato. C- - Colony PCR using distilled water as template. 1-6 - DNA samples from different bacterial colonies were obtained through a pathogen isolation assay performed 24 h after infection. 7-13 - DNA samples from different bacterial colonies were obtained through a pathogen isolation assay performed 48 h after infection. C+ *- Pseudomonas syringae* pv. tomato DNA stored at -80 ºC belonging to the laboratory bacterial collection.

### DETAILED DESCRIPTION

**Figure 1** presents a device, robotic platform and metabolic quantification according to the disclosure. The device comprises a spectroscopy Point-of-Measurement (POM) to record the spectral fingerprint of plant tissues. The POM can record the spectra of the internal structures of plant tissues, allowing the measurement of key metabolites representative of biochemical cycles and regulatory mechanisms, such as pigments, tannins, photosynthesis, energy storage, and nutrient uptake.

In an embodiment, the present disclosure is deployed/integrated on existing systems and devices, thus extending their technical capabilities. It enables the existing system to explore in detail plant internal structures and composition for a new *omics* approach to precision agriculture, where molecular biology and plant physiology are key enablers of new diagnosis and agricultural practices. The disclosure includes, **Figure 2****.** the basic principles for macroscopic tomography reconstruction using latent variables. The method is based in two parts: i. spectral recording of samples at different point-of-view; and ii. establishment of internal structures spectral database.

In an embodiment, the two are related by hierarchical latent structures models in **Figure 2****,** whereby the information of the object's different perspectives are matched against the knowledge base of tissues to provide a reconstruction of internal sample structures from the observed spectra.

A similar strategy is used for hyperspectral microscopy, as exemplified in **Figure 3****.**

**Figure 3** presents an embodiment of hyperspectral microscopy tomography and its relationship to macroscopic measurements: (a) hyperspectral image data of infected leaf along different depths (z) where the extension of the infected lesions is explicit in spectral patterns; and (b) macroscopic measurements with device and corresponding classification of infection using linear discriminant analysis (LDA) to spectral data.

In an embodiment, a scanning hyperspectral microscope was implemented to scan at 200× magnification inoculated tomato leaves, taking the spectra along the x, y and z axis. The 3D dataset is stored in the tensor format, to each space coordinate has a corresponding spectrum. The evolution of spectral fingerprints of infection and control are used to create classification models to be used in both hyperspectral microscopy diagnosis and multi-scaled diagnosis.

Multi-scaled reconstruction is presented in **Figure 4****.** In an embodiment, the macroscopic spectra are recorded using 200 µm fiber optics and are related to the 200× hyperspectral image by their corresponding latent space. The latent space correspondence provides the correspondence between the two spectral pieces of information, as the macroscopic record is a superposition of the microscopic hyperspectral images at each z-plane. In this sense, small changes in the macroscopic spectra can be related to the significant changes in the spectra observed with magnification at the different stages of the disease, enabling an early diagnosis with existing low-cost hardware.

Tomato *(Solanum lycopersicum L.)* plants of the cultivar Cherry were grown in 200 mL pots containing a commercial potting substrate, in a walk-in plant growth chamber under controlled conditions (t= 25-27 ºC, humidity≈ 60%, and photoperiod of 12/12 h). Plants were divided into three groups: control, **Pst** and **Xeu;** control was used as the control (submitted to sterile water treatment), **Pst** was the group for inoculation with *Pseudomonas syringae* pv. tomato, whereas **Xeu** was the group for inoculation with *Xanthomonas euvesicatoria* LMG 905. Plants were inoculated in the laboratory, at the growth stage of 5-6 fully expanded leaves, by liquid aerosolisation until saturation was achieved with subsequent run-off. Differently inoculated plants were segregated from each other, in order to avoid any possible cross-contamination.

The bacterial suspensions used for these inoculation assays consisted of 1 x 10⁸ cells.mL⁻¹ for **Pst** and 2 x 10⁸ cells.mL⁻¹ for **Xeu,** prepared from a 48 h old culture, grown on KB medium (peptone, 20.0 g; glycerol, 10.0 mL; MgSO₄, 1.5 g; K₂PO₄, 1.5 g; agar, 15.0 g; distilled water, up to 1.0 liter), for **Pst,** and YDC medium, for **Xeu** (yeast extract, 10.0 g; dextrose, 20.0 g; CaCO₃, 20.0 g; agar, 15.0 g; distilled water, up to 1.0 liter). Afterwards, the inoculated plants were covered with transparent polythene bags for 48 h in order to increase the relative humidity that fosters bacterial entry into plant tissues, through natural openings, i.e., stomata.

Plants were monitored daily for symptomatic evolution for 7 days. Leaf samples of all groups (control, **Pst** and **Xeu)** were collected once every 24 h, until the 4^{th} day, and one final sampling moment at 144 h, in a total of 5 sampling moments per group.

Simultaneously and in order to verify if the bacteria cultures used in the inoculation assays were viable, 20 µL of **Pst** and **Xeu** solution was cultured in Petri dishes containing KB and YDC media, respectively. After 48 h, it was possible to observe bacterial growth in both nutrient media, supporting the bacterial viability at inoculation.

After the inoculation assays, plant leaves from each group were collected and stored in separate plastic bags. Afterwards, for sample disinfection, the surface of each sample was washed in 70% alcohol for 5 min, followed by rinsing with sterile distilled water for 1 min; this procedure was repeated 5 times. Disinfected samples were placed in different homogenising bags containing a side filter. The samples were grounded in 2 mL of sterile distilled water until full tissue maceration was achieved. The obtained mixture was diluted with sterile distilled water to a final volume of 6 mL and collected after passing the filter bag. Twenty microliters of each sample solution were plated in separate Petri dishes containing KB **(Pst** extracts) and YDC medium **(Xeu** extracts), respectively, and incubated for 48 h at 28 ºC. Bacterial colonies that presented characteristic **Pst** and **Xeu** morphology were isolated and used in colony PCR.

Primer pairs designed by Vieira et al. [7] were selected for PCR validation of leaves infected with Pst. Similarly, infection of leaves infected with Xeu was confirmed by PCR using primer pairs designed by Albuquerque et al. [8]. Polymerase chain reactions were carried out in 20 µL reactions containing 1× DreamTaq Buffer (Thermo Scientific, Vilnius, Lithuania), 0.2 mM of each dNTP (Thermo Scientific), 0.2 µM of each primer (STAB Vida, Lisbon, Portugal), 1 U of DreamTaq DNA Polymerase (Thermo Scientific), and 10 µL of a dilution of bacterial cells in sterile water. The PCR conditions for the confirmation of **Pst** were as follows: an initial denaturation step of 3 min at 95 ºC, followed by 35 cycles of 30 s at 95 ºC, 30 s at 63 ºC and 30 s at 72 ºC with a final extension step of 10 min at 72 ºC [7]. Similarly, PCR conditions for the confirmation of **Xeu** were as follow: an initial denaturation step of 5 min at 95 ºC, followed by 35 cycles of 30 s at 95 ºC, 30 s at 61 ºC and 30 s at 72 ºC with a final extension step of 10 min at 72 ºC [8]. PCR products were separated on 0.8% agarose gels stained with GreenSage Premium (Nzytech, Lisbon, Portugal).

In an embodiment, macroscopic tomography reconstruction is performed by recording the spectra to the whole grape in two different apparatus: i. direct transmittance using the described device; and ii. full transmittance using a power led light source as opposed to the transmittance probe with light collection fibers, using a reflectance probe and HR4000 spectrometer from Ocean Insight. The same grape was afterward separated into its different tissues (skin, pulp, and seeds) for the spectral recording of these grape components. A total of 192 grapes were used to provide statistical support to macroscopic reconstruction.

Leaf samples of each plant were excised of the plant and readily analysed: each leaf was deposited on a single glass slide, supported by a platform featuring a pinhole slit (< 0.5 mm) from which white light was projected (> 65000 lux). The stainless-steel probe was then placed perpendicularly to the leaf (adaxial side) on select spots (3×) throughout each leaf (radial orientation) for spectral acquisition. This configuration minimises light scattering, allowing light collimation towards the fiber within the probe, maintaining a minimal measurement area (200 µm), and providing maximum discrimination by leaf scanning. No further sample preparation was required nor performed.

**Figure 5** presents an embodiment of a macroscopical setup consisting of a manual probe for data acquisition on select matrix spots.

Spectroscopy measurements were performed using UV-Vis-NIR fiber optic (I.D. 200 µm) stainless steel probe connected to an Ocean Insight model HR4000 spectrometer, with dynamic acquisition times, so that spectral data was fully comprehended within the linear range of the spectrometer. Data acquisition was performed within the 200-1100 nm range, and every 24 h, until the 4^{th} day, and one final sampling moment at 144 h, in a total of 5 sampling moments per group.

Leaf samples of each plant were excised of the plant and readily analysed: samples were laid between glass slides, and placed on the microscopical setup for data acquisition. A 5×5 grid (millimetre spacing) sampling grid was devised on each leaf, comprising of 25 data points of spectroscopic data; sampling grid initial coordinates were randomly set, yet assuring the avoidance of the leaf main stem line. No further sample preparation was required nor performed.

The hyperspectral microscopy system, consisting of a Zeiss Axiovert.A1, in transmission mode, was modified and customised to perform 3D hyperspectral microscopy for sequential x, y, z mapping. The following modifications were performed:
i. Ocular adapter for spectral measurements: an adapter was developed to adapt a fiber optics spectrometer (HR4000) to the ocular. The adapter allows aligning the measurement fiber with the center of the microscope camera, allowing to synchronise the spectral measurement with the recorded image at any magnification and position (x, y, and z-axis);
ii. Adapter for direct *in vivo* leaf measurements using two glass slides;
iii. Hyperspectral scanning and imaging software: hyperspectral images were performed by linear scanning of the leaf at each z depth, producing a spectral image at each depth. Tomography is recorded layer-by-layer. Imaging software was developed to show each layer using triangular finite elements for allowing continuous rendering of spectral data.

Microscopical imaging was performed using Best Fit microscopy option for macro (Red, Green, Blue - RGB) images, under the same conditions as for microscopical hyperspectral imaging, every 24 h, until the 4^{th} day, and one final sampling moment at 144 h, in a total of 5 sampling moments per group.

**Figure 6** presents an embodiment of a hyperspectral microscope setup composed by a Zeiss Axiovert.A1 **10,** connected to an Ocean Insight HR4000 spectrometer **30** via an UV-Vis-NIR 200 µm (I.D.) optical fiber **20,** fine-tuned for accurate, simultaneous spectral acquisition of a sampled micrograph.

The basis of information relationship within different scales of spectral measurements is performed by hierarchical relationship of super-space and corresponding sub-spaces. The sub-spaces conserve the latent space variables of the different layers of a hyperspectral microscope image, that is, the features of these images are linear combinations of the vectors spawning the sub-space at a given space coordinate [9,10]. These characteristics are used to derive a super-space of characteristics, translating the combination of a particular hyperspectral image or tomography. The super-space characteristics are afterwards possible to be related to the macroscopic feature space to establish the relationship between macroscopic and microscopic measurements.

Results of the reconstruction of the macroscopic tomography using hierarchical latent variables are presented in **Figure 7****.** In this example, we demonstrate that although the observed spectra in different types of equipment, e.g., robotic device and HR4000 spectrometer, are not similar in terms of their shape, these carry the same information. The two signals' variance space is equivalent. Such means that both are observing the same spectral data but with a different type of spectral fingerprint due to the optical configuration. This fingerprint carries the same physical and chemical information about the observed grapes.

Spectra of the grapes along the maturation follow the displayed parabola of **Figure 7****,** in both variance spaces of the robotic device and HR4000 spectrometer. This parabolic shape is due to the evolution of grape maturation and is also observed in the tomography fusion space, which is obtained by the hierarchical relationship between the latent sub-spaces of the skin, pulp, and seeds tissues spectral data.

The tomographic fusion space is obtained by the information fusion of the variance sub-spaces of spectral characteristics of the skin, pulp, and seeds, obtained by their observed spectra, as seen on **Figure 7****.** The relationship between the robotic device, HR4000, tomography fusion space and the information of the corresponding tissue is bidirectional, i.e., it possible to infer the tissues spectra from the grape spectra - and vice-versa - to estimate the observed grape spectra from the tissue spectra.

This case-study shows how spectral data is correctly represented by its latent structures, spaces, and sub-spaces. The spectral data in tomography, as well as, between different physical scales, is well represented by a hierarchical relationship of the latent structures from each scale. The use of latent structures is highly computationally efficient, as these methods perform all computations in a compressed space, and therefore, as the computational cost is low, the proposed method is ideal for running in low-cost loT (Internet of Things) hardware, allowing the development of very cost-effective hardware/software relation for metabolic tomography.

**Figure 8** exemplifies the reconstruction of the skin, pulp, and seed spectra from the grape spectra recorded by the robotic device from two different grapes collected during veraison and near harvest. At the human eye, these grapes look very similar, and the recorded spectra from the robotic device to the untrained eye show low variation. The grapes are however in very distinct development stages, and their tissues have very significant compositional differences. By using the relationship between the robotic variance space and tomography fusion space, the computed spectra from the skin, pulp, and seed spectra exhibit very significant differences typical of veraison and near harvest development stages. These significant differences correspond to compositional differences in skin pigmentation (chlorophylls vs anthocyanins), sugars and acids accumulated in the pulp tissues, and seed ripeness status (proportional to lignification and tannins) with estimates presented in **Figure 8****.**

Results of tomato leaf bacterial infection diagnosis by *P. syringae* and *X. euvesicatoria* are presented in **Figure 9****.** These show the infection is extremely aggressive to cause damage to the tomato leaf. Even at the first 24 h, spectral analysis shows that chemical composition is already significantly different between the control, *P. syringae* and *X. euvesicatoria.*

At this stage, *P. syringae* exhibits a higher degree of discriminance than *X. euvesicatoria,* when compared to the control samples. As the infection progresses, significant differences between the control and infection agent begin to diverge in terms of spectral patterns and corresponding chemical composition, leading to the isolated classifications of each sample type in the linear discriminant analysis presented in **Figure 9****.** Class separation is obtained for all the sampling times at 24, 48, 72, 96 and 144 hours. Such allows speculating that the infection mechanisms and relationship between an infectious agent and plant response to the infection are characteristic of a particular type of bacteria.

At the naked eye or RGB camera, visible superficial damage appears only after 72 hours after inoculation. Therefore, human or machine vision algorithms (e.g., Deep Learning) will not be able to diagnose the disease before it enters this late stage. Ground truth polymerase chain reaction (PCR) tests shown positive identifications of the presence of *P. syringae* and *X. euvesicatoria* after 24 h of inoculation, as shown in **Figures 11** and **12****.** The PCR method comprises the amplification of bacterial counts by growth media, during 24 to 48 h at 28 ºC, not allowing this molecular biology method to be used as rapid response diagnosis. The presented results of the macroscopic tomography case study shows not only a disruptive way of non-invasive disease diagnosis in plants but also the capacity to provide a very early diagnosis.

Results for hyperspectral microscopy tomography are presented in **Figure 10****.** This figure presents the spectra of hyperspectral tomography of control and infected leaves with *P. syringae* and *X. euvesicatoria* at 144 h after inoculation. At this disease stage, spectral variance can be spawned into 3 principal components, which can classify any of the spectra of each category **(control, Pst** or **Xeu).** *Pseudomonas syringae* shows higher degrees of damage, whereas the spectra patterns are consistent with necrosis. *Xanthomonas euvesicatoria* at this stage has more uniform damage over the hyperspectral data. The spectral patterns from *P. syringae* or *X. euvesicatoria* are distinguishable in the 3PC from each other and control samples. It is also shown the estimated probability of infection by each bacteria at each node of the hyperspectral image using a partial least squares discriminant analysis model. Despite the spectral variance being extremely significant at each hyperspectral image, due to both leaf structures and compositional differences, these do not affect classification performance by a linear classifier. For example, the PLS-DA model correctly predicts the control sample with an extremely low probability of infection and extremely high probabilities of infection for *P. syringae* and *X. euvesicatoria.* These results are in agreement to the microscopy images, where at 144 h all tissues are infected, not existing non-infected regions. The leaf infected by *P. syringae* show already advanced necrosis in some spots, and *X. euvesicatoria* very significant tissue damages. Results also demonstrate that microscopic diagnosis has the potential for early-stage diagnosis, confirming the results obtained by macroscopic tomography.

**Table 1. Benchmark between PCR, Image analysis and information according to the disclosure.**

| | **PCR** | **Image Analysis** | **Present disclosure** |
|---|---|---|---|
| **Type** | Genetic | Pattern Recognition | Spectroscopy Analysis |
| **Information** | Qualitative | Classification | Quantitative and Qualitative |
| ***In vivo*** | No | Yes | Yes |
| **Metabolites** | No | No | Yes |

**Figure 11** presents an embodiment of colony PCR analysis of infected tomato leaves with DNA markers XV14 (713 bp) for the detection *of Xanthomonas euvesicatoria* LMG 905. C- - Colony PCR using distilled water as template. 1, 2, 3 - DNA samples from different bacterial colonies were obtained through a pathogen isolation assay performed 24 h after infection. 4, 5 - DNA samples from different bacterial colonies obtained through pathogen isolation assay performed 48 h after infection. C+ - *Xanthomonas euvesicatoria* DNA stored at -80 ºC belonging to the laboratorial bacterial collection.

**Figure 12** presents an embodiment of colony PCR analysis of infected tomato leaves with DNA markers PST2 (200 bp) for detection of *Pseudomonas syringae* pv. tomato. C- - Colony PCR using distilled water as template. 1-6 - DNA samples from different bacterial colonies obtained through pathogen isolation assay performed 24 h after infection. 7-13 - DNA samples from different bacterial colonies obtained through pathogen isolation assay performed 48 h after infection. C+ *- Pseudomonas syringae* pv. tomato DNA stored at -80 ºC belonging to the laboratorial bacterial collection.

To our best knowledge, the state-of-the-art does not provide a tool similar to the present disclosure. Results show that the disclosed technology has high potential to revolutionise precision agriculture in its dimensions by being able to monitor the omics of each plant in vast plantations, providing information for the study of the complex relationship of plant-soil-climate-ecosystem and agricultural practices.

The benchmarks presented in **Table 1** show how this type of technology can improve precision agriculture's state of the art. By focusing precision agriculture on plant physiology and its molecular biology, new horizons can be achieved by: i. developing new agricultural practices with omics scientific evidence; ii. introducing a modern way of developing agricultural practices by the use of *in silico* models from bioinformatics and systems biology; iii. linking plant biotechnology molecular biology *in vitro* results towards *in vivo* field applications; and iv. understanding how the physiology of plants inserted into a natural ecosystem responds when compared to laboratory observations.

Flow diagrams of particular embodiments of the presently disclosed methods are depicted in figures. The flow diagrams illustrate the functional information one of ordinary skill in the art requires to perform said methods required in accordance with the present disclosure.

It will be appreciated by those of ordinary skill in the art that unless otherwise indicated herein, the particular sequence of steps described is illustrative only and can be varied without departing from the disclosure. Thus, unless otherwise stated, the steps described are so unordered meaning that, when possible, the steps can be performed in any convenient or desirable order.

It is to be appreciated that certain embodiments of the disclosure as described herein may be incorporated as code (e.g., a software algorithm or program) residing in firmware and/or on computer useable medium having control logic for enabling execution on a computer system having a computer processor, such as any of the servers described herein. Such a computer system typically includes memory storage configured to provide output from execution of the code which configures a processor in accordance with the execution. The code can be arranged as firmware or software and can be organised as a set of modules, including the various modules and algorithms described herein, such as discrete code modules, function calls, procedure calls or objects in an object-oriented programming environment. If implemented using modules, the code can comprise a single module or a plurality of modules that operate in cooperation with one another to configure the machine in which it is executed to perform the associated functions, as described herein.

The disclosure should not be seen in any way restricted to the embodiments described and a person with ordinary skill in the art will foresee many possibilities to modifications thereof. The above-described embodiments are combinable. The following claims further set out particular embodiments of the disclosure.

It is acknowledged the contribution of Fundação Amadeus Dias, Santander Universidades, and the University of Porto in respect of the BIP PROOF 2021 AWARD.

This work was supported by Project "OmicBots: High-Throughput Integrative Omic-Robots Platform for a Next Generation Physiology-based Precision Viticulture/PTDC/ASP-HOR/1338/2021", financed by the National Funds through the FCT - Fundação para a Ciência e a Tecnologia, I.P. (Portuguese Foundation for Science and Technology).

### References

[1] S. Arshavsky-Graham, E. Segal, Lab-on-a-Chip devices for point-of-care medical diagnostics. Advances in Biochemical Engineering/Biotechnology, Springer, Berlin, Heidelberg, 2020, https://doi.org/10.1007/10_2020_127.
[2] S. Nishat, A.T. Jafry, A.W. Martinez, F.R. Awan, Paper-based microfluidics: simplified fabrication and assay methods, Sens. Actuators B Chem. 336 (2021) 129681, https://doi.org/10.1016/j.snb.2021.129681
[3] T. Zhou, D. Lu, Q. She, C. Chen, J. Chen, Z. Huang, S. Feng, R. You, Y. Lu, Hypersensitive detection of IL-6 on SERS substrate calibrated by dual model, Sens. Actuators B Chem. 336 (2021) 129597, https://doi.org/10.1016/j.snb.2021.129597.
[4] K. Jiang, J. Wu, Y. Qiu, Y.Y. Go, K. Ban, H.J. Park, J.H. Lee, Plasmonic colorimetric PCR for Rapid molecular diagnostic assays, Sens. Actuators B Chem. 337 (2021) 129762, https://doi.org/10.1016/j.snb.2021.129762
[5] J. Prakash, A. Dey, S. Uppal, R. Alexander, A. Kaushal, H.S. Misra, K. Dasgupta, Label-free rapid electrochemical detection of DNA hybridisation using ultrasensitive standalone CNT aerogel biosensor, Biosensors and Bioelectronics, 191 (2021) 113480, https://doi.org/10.1016/j.bios.2021.113480.
[6] J. Liu, X. Wang Plant diseases and pests detection based on deep learning: a review. Plant Methods 17, 22 (2021). https://doi.org/10.1186/s13007-021-00722-9
[7] J. Vieira, M.V. Mendes, P. Albuquerque, P. Moradas-Ferreira, F. Tavares, A novel approach for the identification of bacterial taxa-specific molecular markers. Lett Appl Microbiol. 44 (2007), 506-12. https://doi.org/10.1111/j.1472-765X.2007.02109.x.
[8] P. Albuquerque, C.M.R. Caridade, A.S. Rodrigues, A.R.S. Marcal, J. Cruz, et al., Evolutionary and Experimental Assessment of Novel Markers for Detection of Xanthomonas euvesicatoria in Plant Samples. PLOS ONE (2012) 7, e37836. https://doi.org/10.1371/journal.pone.0037836
[9] J.A. Westerhuis, T. Kourti, and J.F. MacGregor, Analysis of multiblock and hierarchical PCA and PLS models. J. Chemometrics, 12 (1998), 301-321. https://doi.org/10.1002/(SICI)1099-128X(199809/10)12:5<301::AID-CEM515>3.0.CO;2-S
[10] O.E. de Noord, Theobald E.H., Multilevel component analysis and multilevel PLS of chemical process data. J. Chemometrics, 19 (2005) 301-307. https://doi.org/10.1002/cem.933
[11] J. Chan, S.C. Raju, E. Topol. Towards a tricorder for diagnosing paediatric conditions. The Lancet (British Edition), 394 (2019) 10202, 907-907.
[12] H. Waters, New $10 million X prize launched for tricorder-style medical device. Nature Medicine, 17 (2011) 754-754

## Claims

1. Computer-implemented method for non-invasive tomographic metabolite characterisation of a vegetable or animal sample to be characterised, the sample comprising a plurality of differentiated tissues from a set of previously-characterised differentiated tissues, by using a multidimensional latent structure model of differentiated tissues, which was previously obtained by:
scanning tissue samples belonging to each differentiated tissue of the set with a hyperspectral microscope and spectrometer;
acquiring hyperspectral spectrum or spectra for each differentiated tissue sample from the hyperspectral microscope and spectrometer;
analysing the same tissue samples for the metabolites to be characterised;
calculating the latent structure model from the acquired spectra and analysed metabolites;
wherein the method comprises the steps of:
spatially scanning the sample to be characterised with a hyperspectral point-of-measurement, POM, optical device and a spectrometer;
acquiring hyperspectral spectrum or spectra from each of a plurality of spatial positions and/or orientations of the POM optical device and spectrometer in respect of the sample to be characterised;
using the previously obtained model to deconvolute the metabolites to be characterised for each of the differentiated tissues from the acquired spectra;
wherein the multidimensional latent structure model is a hierarchical latent structure model;
wherein the hierarchical latent structure model is comprised of a latent variable model comprising a latent variable super-space and corresponding latent variable sub-spaces; wherein the latent variable super-space is a super-space for each of the metabolites to be characterised, translating the combination of hyperspectral data to the metabolites to be characterised;
wherein each of the latent variable sub-spaces is a latent sub-space for spectral data of each of the differentiated tissues.

2. Method according to the previous claim wherein the scanning tissue samples belonging to each tissue of the set with a hyperspectral microscope and spectrometer is microscopic, and
the spatially scanning the sample to be characterised with a hyperspectral POM optical device and spectrometer is macroscopic.

3. Method according to the previous claim wherein spatially scanning comprises varying one or more of the following to obtain a spatial diversity of the POM spatial scanning:
displacement of the POM optical device;
point-of-view angle of the POM optical device;
different scales of magnification of the POM optical device;
moving the POM optical device lines of sight to cause a parallax effect.

4. Method according to any of the previous claims wherein the sample to be characterised is a three-dimensional tissue structure or organ.

5. Method according to any of the previous claims wherein the metabolites to be characterised comprise pigments, tannins, photosynthesis, energy storage, nutrient uptake, necrosis, infection probability, or combinations thereof.

6. Method according to any of the previous claims wherein the sample to be characterised is a vegetable sample and the differentiated tissues comprise skin, pulp and seed tissue.

7. Method according to the previous claim wherein the sample to be characterised is a vegetable sample, in particular the sample to be characterised is a tomato or grape tissue sample.

8. Method according to any of the previous claims wherein the scanning of tissue samples belonging to each differentiated tissue is carried out along a sampling grid.

9. Method according to any of the previous claims wherein the scanning of tissue samples belonging to each differentiated tissue is carried out along discrete sampling depths.

10. Method according to any of the previous claims wherein the spectra are UV-VIS-NIR.

11. Device for non-invasive tomographic metabolite characterisation of a vegetable or animal sample to be characterised, the sample comprising a plurality of differentiated tissues from a set of previously-characterised differentiated tissues, comprising a computer processor and a non-transitory computer-readable medium comprising a multidimensional latent structure model of differentiated tissues, which was previously obtained by:
scanning tissue samples belonging to each differentiated tissue of the set with a hyperspectral microscope and spectrometer;
acquiring hyperspectral spectrum or spectra for each differentiated tissue sample from the hyperspectral microscope and spectrometer;
analysing the same tissue samples for the metabolites to be characterised;
calculating the latent structure model from the acquired spectra and analysed metabolites;
wherein the computer processor is configured for:
spatially scanning the sample to be characterised with a hyperspectral point-of-measurement, POM, optical device and a spectrometer;
acquiring hyperspectral spectrum or spectra from each of a plurality of spatial positions and/or orientations of the POM optical device and spectrometer in respect of the sample to be characterised;
using the previously obtained model to deconvolute the metabolites to be characterised for each of the differentiated tissues from the acquired spectra;
wherein the multidimensional latent structure model is a hierarchical latent structure model;
wherein the hierarchical latent structure model is comprised of a latent variable model comprising a latent variable super-space and corresponding latent variable sub-spaces;
wherein the latent variable super-space is a super-space for each of the metabolites to be characterised, translating the combination of hyperspectral data to the metabolites to be characterised;
wherein each of the latent variable sub-spaces is a latent sub-space for spectral data of each of the differentiated tissues.

12. Non-transitory computer-readable medium comprising computer program instructions for implementing a device for non-invasive tomographic metabolite characterisation of a vegetable or animal sample to be characterised, which when executed by a processor, cause the processor to carry out the method of any of the claims 1-10.

## Patentansprüche

1. Computerimplementiertes Verfahren zur nichtinvasiven tomographischen Charakterisierung von Metaboliten einer zu charakterisierenden pflanzlichen oder tierischen Probe, wobei die Probe eine Vielzahl differenzierter Gewebe aus einem Satz von im Voraus charakterisierten differenzierten Geweben umfasst, unter Verwendung eines mehrdimensionalen latenten Strukturmodells differenzierter Gewebe, das im Voraus erhalten wurde durch:
Abtasten von Gewebeproben, die zu dem jeweiligen differenzierten Gewebe des Satzes gehören, mit einem hyperspektralen Mikroskop und Spektrometer;
Erfassen des hyperspektralen Spektrums bzw. der Spektren für jede differenzierte Gewebeprobe des hyperspektralen Mikroskops und dem Spektrometer;
Untersuchen derselben Gewebeproben der zu charakterisierenden Metaboliten;
Berechnen des latenten Strukturmodells aus den erfassten Spektren und untersuchten Metaboliten;
wobei das Verfahren die folgenden Schritte umfasst:
Räumliches Abtasten der zu charakterisierenden Probe mit einem hyperspektralen Messpunkt (POM), einer optischen Vorrichtung und einem Spektrometer;
Erfassen des hyperspektralen Spektrums bzw. der Spektren von jeder der einen Vielzahl räumlicher Positionen und/oder Ausrichtungen der optischen POM-Vorrichtung und des Spektrometers in Bezug auf die zu charakterisierende Probe;
Verwenden des im Voraus erhaltenen Modells zum Dekonvolieren der zu charakterisierenden Metaboliten für jedes der differenzierten Gewebe aus den erfassten Spektren;
wobei das mehrdimensionale latente Strukturmodell ein hierarchisches latentes Strukturmodell ist;
wobei das hierarchisch latente Strukturmodell aus einem latenten Variablenmodell besteht, umfassend einen übergeordneten latenten Variablenraum und entsprechende latente Teilvariablenräume; wobei der übergeordnete latente Variablenraum ein übergeordneter Raum für jeden der zu charakterisierenden Metaboliten ist, der die Kombination der Hyperspektraldaten auf die zu charakterisierenden Metaboliten überträgt;
wobei jeder der latente Teilvariablenräume ein latenter Teilraum für Spektraldaten jedes der differenzierten Gewebe ist.

2. Verfahren nach dem vorangehenden Anspruch, wobei das Abtasten der mit einem hyperspektralen Mikroskop und Spektrometer abzutastenden Gewebeproben, die jeweils zu einem Satz gehören, mikroskopisch erfolgt, und
das räumliche Abtasten der mit einer hyperspektralen optischen POM-Vorrichtung und einem Spektrometer der zu charakterisierenden Probe makroskopisch erfolgt.

3. Verfahren nach dem vorangehenden Anspruch, wobei das räumliche Abtasten das Variieren eines oder mehrerer der folgenden Punkte umfasst, um eine räumliche Vielfalt des räumlichen POM-Abtastens zu erreichen:
Verschieben der optischen POM-Vorrichtung;
Ausrichtungswinkel der optischen POM-Vorrichtung;
verschiedene Vergrößerungsmaßstäbe der optischen POM-Vorrichtung;
Bewegen der Sichtlinien der optischen POM-Vorrichtung, um einen Parallaxeneffekt zu erzeugen.

4. Verfahren nach einem der vorangehenden Ansprüche, wobei die zu charakterisierende Probe eine dreidimensionale Gewebestruktur oder ein Organ ist.

5. Verfahren nach einem der vorangehenden Ansprüche, wobei die zu charakterisierenden Metaboliten Pigmente, Tannine, Photosynthese, Energiespeicher, Nährstoffaufnahme, Nekrose, Infektionswahrscheinlichkeit oder Kombinationen davon umfassen.

6. Verfahren nach einem der vorangehenden Ansprüche, wobei die zu charakterisierende Probe eine pflanzliche Probe ist und die differenzierten Gewebe Haut, Fruchtfleisch und Samengewebe umfassen.

7. Verfahren nach dem vorangehenden Anspruch, wobei die zu charakterisierende Probe eine pflanzliche Probe ist, wobei die zu charakterisierende Probe insbesondere eine Gewebeprobe von Tomaten oder Trauben ist.

8. Verfahren nach einem der vorangehenden Ansprüche, bei dem die zu jedem der einzelnen Gewebe gehörenden Gewebeproben entlang eines Abtastgitters abgetastet werden.

9. Verfahren nach einem der vorangehenden Ansprüche, wobei das Abtasten der Gewebeproben, die zu jedem differenzierten Gewebe gehören, entlang einzelner Tiefen für die Probenahme erfolgt.

10. Verfahren nach einem der vorangehenden Ansprüche, wobei die Spektren UV-VIS-NIR sind.

11. Vorrichtung zur nichtinvasiven tomographischen Charakterisierung von Metaboliten einer zu charakterisierenden pflanzlichen oder tierischen Probe, wobei die Probe eine Vielzahl differenzierter Geweben aus einem Satz von im Voraus charakterisierten differenzierten Geweben umfasst, umfassend einen Computerprozessor und ein nichtflüchtiges computerlesbares Medium, umfassend ein mehrdimensionales latentes Strukturmodell differenzierter Gewebe, das im Voraus erhalten wurde durch:
Abtasten von Gewebeproben, die zu dem jeweiligen differenzierten Gewebe des Satzes gehören, mit einem hyperspektralen Mikroskop und Spektrometer;
Erfassen des hyperspektralen Spektrums bzw. der Spektren für jede differenzierte Gewebeprobe des hyperspektralen Mikroskops und dem Spektrometer;
Untersuchen derselben Gewebeproben der zu charakterisierenden Metaboliten;
Berechnen des latenten Strukturmodells aus den erfassten Spektren und untersuchten Metaboliten;
wobei der Computerprozessor konfiguriert ist für:
Räumliches Abtasten der zu charakterisierenden Probe mit einem hyperspektralen Messpunkt (POM), einer optischen Vorrichtung und einem Spektrometer;
Erfassen des hyperspektralen Spektrums bzw. der Spektren von jeder der einen Vielzahl räumlicher Positionen und/oder Ausrichtungen der optischen POM-Vorrichtung und des Spektrometers in Bezug auf die zu charakterisierende Probe;
Verwenden des im Voraus erhaltenen Modells zum Dekonvolieren der zu charakterisierenden Metaboliten für jedes der differenzierten Gewebe aus den erfassten Spektren;
wobei das mehrdimensionale latente Strukturmodell ein hierarchisches latentes Strukturmodell ist;
wobei das hierarchische latente Strukturmodell aus einem latenten Variablenmodell besteht, das einen übergeordneten latenten Variablenraum und entsprechende Teilvariablenräume umfasst;
wobei der übergeordnete latente Variablenraum ein übergeordneter Raum für jeden der zu charakterisierenden Metaboliten ist, der die Kombination der Hyperspektraldaten auf die zu charakterisierenden Metaboliten überträgt;
wobei jeder der latenten Teilvariablenräume ein latenter Teilraum für Spektraldaten jedes der differenzierten Gewebe ist.

12. Nichtflüchtiges computerlesbares Medium, umfassend Computerprogrammanweisungen zum Implementieren einer Vorrichtung zur nichtinvasiven tomographischen Charakterisierung von Metaboliten einer zu charakterisierenden pflanzlichen oder tierischen Probe, die, wenn sie von einem Prozessor ausgeführt werden, den Prozessor veranlassen, das Verfahren gemäß einem der Ansprüche 1-10 durchzuführen.

## Revendications

1. Procédé mis en œuvre par ordinateur pour la caractérisation tomographique non invasive de métabolites d'un échantillon végétal ou animal devant être caractérisé, l'échantillon comprenant une pluralité de tissus différenciés issus d'un ensemble de tissus différenciés préalablement caractérisés, en utilisant un modèle de structure latent multidimensionnel de tissus différenciés, qui a été préalablement obtenu par :
balayage d'échantillons de tissus appartenant à chacun des tissus différenciés de l'ensemble avec un microscope hyperspectral et un spectromètre ;
acquisition de spectre ou spectres pour chaque échantillon de tissus différenciés à partir du microscope hyperspectral et du spectromètre ;
analyse des mêmes échantillons de tissus pour que les métabolites soient caractérisés ;
calcul du modèle de structure latent à partir des spectres acquis et des métabolites analysés ;
dans lequel le procédé comprend les étapes consistant à :
effectuer le balayage spatial de l'échantillon devant être caractérisé avec un dispositif optique de point de mesure, POM, hyperspectral et un spectromètre ;
acquérir le spectre ou les spectres hyperspectral(aux) à partir de chacune d'une pluralité de positions et/ou orientations spatiales du dispositif optique POM et du spectromètre concernant l'échantillon devant être caractérisé ;
utiliser le modèle préalablement obtenu pour déconvoluer les métabolites devant être caractérisés pour chacun des tissus différenciés des spectres acquis ;
dans lequel le modèle de structure latent multidimensionnel est un modèle de structure latent hiérarchique ;
dans lequel le modèle de structure latent hiérarchique comporte un modèle variable latent comprenant un super-espace variable latent et les sous-espaces variables latents correspondants ; dans lequel le super-espace variable latent est un super-espace pour chacun des métabolites devant être caractérisés, traduisant la combinaison de données hyperspectrales aux métabolites devant être caractérisés ;
dans lequel chacun des sous-espaces variables latents est un sous-espace latent pour des données spectrales de chacun des tissus différenciés.

2. Procédé selon la revendication précédente dans lequel le balayage des échantillons de tissus appartenant à chaque tissu de l'ensemble avec un microscope hyperspectral et un spectromètre est microscopique, et
le balayage spatial de l'échantillon devant être caractérisé avec un dispositif optique POM hyperspectral et un spectromètre est macroscopique.

3. Procédé selon la revendication précédente dans lequel le balayage spatial comprend varier un ou plusieurs des éléments suivants pour obtenir une diversité spatiale du balayage spatial POM :
déplacement du dispositif optique POM ;
angle de point de vue du dispositif optique POM ;
différentes échelles d'agrandissement du dispositif optique POM ;
déplacement des lignes de visée du dispositif optique POM pour causer un effet de parallaxe.

4. Procédé selon l'une quelconque des revendications précédentes dans lequel l'échantillon devant être caractérisé est une structure de tissus ou organe tridimensionnel.

5. Procédé selon l'une quelconque des revendications précédentes dans lequel les métabolites devant être caractérisés comprennent les pigments, les tanins, la photosynthèse, le stockage d'énergie, l'absorption de nutriments, la nécrose, la probabilité d'infection, ou des combinaisons de ceux-ci.

6. Procédé selon l'une quelconque des revendications précédentes dans lequel l'échantillon devant être caractérisé est un échantillon végétal et les tissus différenciés comprennent du tissu de la peau, de la pulpe et des graines.

7. Procédé selon la revendication précédente dans lequel l'échantillon devant être caractérisé est un échantillon végétal, l'échantillon devant être caractérisé étant en particulier un échantillon de tissu de tomate ou de raisin.

8. Procédé selon l'une quelconque des revendications précédentes dans lequel le balayage d'échantillons de tissus appartenant à chacun des tissus différenciés est exécuté le long d'une grille d'échantillonnage.

9. Procédé selon l'une quelconque des revendications précédentes dans lequel le balayage d'échantillons de tissus appartenant à chacun des tissus différenciés est exécuté le long de profondeurs d'échantillonnage discrètes.

10. Procédé selon l'une quelconque des revendications précédentes dans lequel les spectres sont UV-VIS-NIR.

11. Dispositif pour la caractérisation tomographique non invasive de métabolites d'un échantillon végétal ou animal devant être caractérisé, l'échantillon comprenant une pluralité de tissus différenciés issus d'un ensemble de tissus différenciés préalablement caractérisés, comprenant un processeur d'ordinateur et un moyen lisible par ordinateur non transitoire comprenant un modèle de structure latent multidimensionnel de tissus différenciés, qui a été préalablement obtenu par :
balayage d'échantillons de tissus appartenant à chacun des tissus différenciés de l'ensemble avec un microscope hyperspectral et un spectromètre ;
acquisition de spectre ou spectres pour chaque échantillon de tissus différenciés à partir du microscope hyperspectral et du spectromètre ;
analyse des mêmes échantillons de tissus pour les métabolites devant être caractérisés ;
calcul du modèle de structure latent à partir des spectres acquis et des métabolites analysés ;
dans lequel le processeur d'ordinateur est configuré pour :
effectuer le balayage spatial de l'échantillon devant être caractérisé avec un dispositif optique de point de mesure, POM, hyperspectral et un spectromètre ;
acquérir le spectre ou les spectres hyperspectral(aux) à partir de chacune d'une pluralité de positions et/ou orientations spatiales du dispositif optique POM et du spectromètre concernant l'échantillon devant être caractérisé ;
utiliser le modèle préalablement obtenu pour déconvoluer les métabolites devant être caractérisés pour chacun des tissus différenciés des spectres acquis ;
dans lequel le modèle de structure latent multidimensionnel est un modèle de structure latent hiérarchique ;
dans lequel le modèle de structure latent hiérarchique comprend un modèle variable latent comprenant un super-espace variable latent et les sous-espaces variables latents correspondants ;
dans lequel le super-espace variable latent est un super-espace pour chacun des métabolites devant être caractérisés, traduisant la combinaison de données hyperspectrales aux métabolites devant être caractérisés ;
dans lequel chacun des sous-espaces variables latents est un sous-espace latent pour des données spectrales de chacun des tissus différenciés.

12. Un moyen lisible par ordinateur non transitoire comprenant des instructions de logiciel informatique destinées à mettre en place un dispositif pour la caractérisation tomographique non invasive de métabolites d'un échantillon végétal ou animal devant être caractérisé qui, lorsqu'il est exécuté par un processeur, fait en sorte que le processeur exécute le procédé de l'une quelconque des revendications 1-10.
